# EUROPÄISCHE PATENTSCHRIFT

(11) **EP 2 457 556 B1**
(45) Veröffentlichungstag und Bekanntmachung des Hinweises auf die Patenterteilung: **18.07.2018**
(21) Anmeldenummer: 11190312.6
(22) Anmeldetag: 23.11.2011
(51) Int. Cl.: A61K 8/34, A61K 8/37, A61K 8/41, A61K 8/42, A61K 8/44, A61K 8/46, A61K 8/60, A61K 8/9711, A61Q 5/02, A61Q 5/12, A61Q 19/10

(54) **Haarbehandlungsmittel**
Hair treatment agent
Produit de traitement des cheveux

(30) Priorität: 30.11.2010 DE 102010062155
(43) Veröffentlichungstag der Anmeldung: 30.05.2012
(73) Patentinhaber: Henkel AG & Co. KGaA, 40589 Düsseldorf (DE)
(72) Erfinder: Scheunemann, Volker, 21339 Lüneburg (DE); Schulze Zur Wiesche, Erik, 20144 Hamburg (DE); Holtkötter, Olaf, 50354 Hürth (DE)

(56) Entgegenhaltungen:
- WO-A1-02/47644
- WO-A1-2004/054533
- WO-A2-2008/039515
- DE-C1- 4 233 504
- KR-A- 20090 096 256
- KR-A- 20090 127 740
- "SACCHAROMYCES/LAMINARIA SACCHARINA FERMENT", INCI Directory , 27. Februar 2009 (2009-02-27), XP002676143, Gefunden im Internet: URL:http://www.specialchem4cosmetics.com/s ervices/inci/ingredient.aspx?id=12086 [gefunden am 2012-05-12]
- DATABASE WPI Week 198432 Thomson Scientific, London, GB; AN 1984-198105 XP002676144, & JP 59 112912 A (FUJI KOSAN KK) 29. Juni 1984 (1984-06-29)
- DATABASE WPI Week 198431 Thomson Scientific, London, GB; AN 1984-192825 XP002676145, & JP 59 110608 A (FUJI KOSAN KK) 26. Juni 1984 (1984-06-26)
- EINAR MOEN ET AL: "Aerobic microbial degradation of alginate in Laminaria hyperborea stipes containing different levels of polyphenols", JOURNAL OF APPLIED PHYCOLOGY, KLUWER ACADEMIC PUBLISHERS, DO, Bd. 9, Nr. 1, 1. Januar 1997 (1997-01-01), Seiten 45-54, XP019247845, ISSN: 1573-5176
- "Die neue Generation Algenkosmetik", Oceanbasis GmbH , XP002676146, Gefunden im Internet: URL:http://www.oceanwell.de/inhalt-wirksto ff/inhaltsstoffe/ [gefunden am 2012-05-16]

## Beschreibung

Die Erfindung liegt auf dem Gebiet der Kosmetik und betrifft Haarbehandlungsmittel, die neben speziellen Tensiden einen Extrakt natürlich fermentierter Braunalgen enthalten.

Die Erfindung betrifft weiterhin die Verwendung der Haarbehandlungsmittel zur Beruhigung empfindlicher Kopfhaut während der Haarreinigung und/oder der Haarpflege.

Bedingt durch das steigende Hygienebewusstsein sowie die Bekämpfung unerwünschter haptischer oder optischer Eigenschaften werden menschliche Haare üblicherweise einer Vielzahl kosmetischer Behandlungen unterzogen.

Diese beginnen mit der täglichen Haarreinigung und führen über die Haarkonditionierung zur Behandlung mit Styling-, Färbe- und/oder Haarverformungsmitteln.

Jede Behandlung erfordert die Verwendung eines anderen Mittels, so dass die Haare mit einer Vielzahl von Wirkstoffen in Kontakt gebracht werden.

Die häufige Wiederholung der Haarbehandlungen kann (insbesondere bei sensibler Kopfhaut) zu einer negativen Beeinflussung der Kopfhaut führen.

Sichtbare Anzeichen davon sind beispielsweise gereizte, trockene, raue, rissige, gerötete, schuppige und sogar entzündete Kopfhaut.

Zur Linderung, Vermeidung und/oder Beseitigung sensibilisierter Kopfhaut sowie aus Zeit- und Umweltgesichtspunkten werden von den Verbrauchern deshalb zunehmend Haarbehandlungsmittel gefordert, mit denen mehrere der zuvor genannten Haarbehandlungen in einem Behandlungsschritt durchgeführt werden können.

Des Weiteren werden von den Verbrauchern Haarbehandlungsmittel auf der Basis kosmetischer Rohstoffe gewünscht, die aus natürlichen Quellen stammen (den so genannten nachwachsenden Rohstoffen).

Die Herstellung solcher Kombinationsprodukte ist jedoch meist aufwendig, denn die für die jeweilige Applikationsform erforderlichen Wirkstoffe (auch der Wirkstoffe natürlichen Ursprungs) sind nicht immer kompatibel und können die Formulierungen destabilisieren.

Es besteht weiterhin der Bedarf nach vorwiegend aus natürlichen Quellen stammenden kosmetischen Rohstoffen, die sich problemlos in Haarbehandlungsmittel einarbeiten lassen, und vorzugsweise über eine "Breitbandwirkung" verfügen, d.h. unterschiedliche kosmetische Probleme gleichzeitig lösen.

Der vorliegenden Erfindung lag die Aufgabe zugrunde, Haarbehandlungsmittel bereitzustellen, die ihrem jeweiligen Zweck entsprechend (Reinigung, Konditionierung, Styling, Färbung, Verformung) hocheffektiv sind und den behandelten Haaren Glanz, Glätte, Volumen und Weichheit verleihen, ohne die Kopfhaut negativ zu beeinflussen.

Eine weitere Aufgabe der vorliegenden Erfindung bestand darin kosmetische Rohstoffe natürlichen Ursprungs zu finden, die sich problemlos in die Haarbehandlungsmittel einarbeiten lassen, und eine Vielzahl der zuvor genannten Anforderungen erfüllen.

Ein weiteres Ziel war die Herstellung reiz- und sensibilisierungsarmer Haarbehandlungsmittel, die die Kopfhaut beruhigen.

Gegenstand der Erfindung sind in einer ersten Ausführungsform Haarbehandlungsmittel, die in einem kosmetischen Träger
a) 0,1 bis 40 Gew.-% mindestens eines Tensids, ausgewählt aus der Gruppe der anionischen, amphoteren/zwitterionischen und/oder kationischen Tenside, und
b) einen Extrakt natürlich fermentierter Braunalgen *Laminaria Saccharina (Zuckertang),* der durch natürliche (alkoholische) Gärung mit einem Pilz erhältlich ist, wobei sich die Mengenangaben auf das Gesamtgewicht des Haarbehandlungsmittels beziehen. Die erfindungsgemäßen Haarbehandlungsmittel enthalten die zwingenden Komponenten in einem kosmetischen Träger, der bevorzugt wässrig oder wässrig-alkoholisch ist.

Bevorzugt enthält der kosmetische Träger mindestens 50 Gew.-% Wasser.

Weiterhin kann der kosmetische Träger 0,01 bis 40 Gew.-%, bevorzugt 0,05 bis 30 Gew.-% und insbesondere 0,1 bis 20 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Sorbitol, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Die erfindungsgemäßen Haarbehandlungsmittel können als Shampoo, Spülung, Haarkur, Haartonic, Haarspray, Schaumaerosol, Haarwachs, Haargel, Haarfärbemittel, Blondiermittel und/oder Haarverformungsmittel konfektioniert werden, und prinzipiell alle für diese Konfektionierungsformen üblichen Inhaltsstoffe enthalten.

Bevorzugt handelt es sich bei dem erfindungsgemäßen Haarbehandlungsmittel um ein Haarreinigungs- und/oder Haarkonditioniermittel.

Als erste zwingende Komponente enthalten die erfindungsgemäßen Haarbehandlungsmittel (bezogen auf ihr Gesamtgewicht) 0,1 bis 40 Gew.-% mindestens eines Tensids, ausgewählt aus der Gruppe der anionischen, amphoteren/zwitterionischen und/oder kationischen Tenside. Anionische Tenside und/oder amphotere/zwitterionische Tenside werden bevorzugt in Haarreinigungsmitteln eingesetzt.

Zur Bereitstellung besonders milder Haarreinigungsmittel und zur Schonung der Kopfhaut während der Reinigung ist es bevorzugt, dass erfindungsgemäße Haarbehandlungsmittel, welche als Haarreinigungsmittel konfektioniert werden, eine Mischung aus milden anionischen und milden amphoteren/zwitterionischen Tensiden enthalten.

Geeignete anionische Tenside werden den erfindungsgemäßen Haarbehandlungsmitteln - bezogen auf deren Gesamtgewicht - bevorzugt in Mengen von 0,5 bis 25 Gew.-%, mehr bevorzugt von 1 bis 20 Gew.-%, besonders bevorzugt von 2 bis 17,5 Gew.-% und insbesondere von 3 bis 15 Gew.-% zugegeben.

Zu den geeigneten anionischen Tensiden, die in den erfindungsgemäßen Haarbehandlungsmitteln eingesetzt werden können, zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 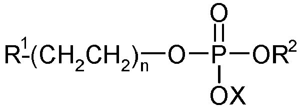
in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁴ unabhängig voneinander stehend für einen C₁ bis C₄ - Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.

Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Geeignete amphotere/zwitterionische Tenside können den erfindungsgemäßen Haarbehandlungsmitteln - bezogen auf deren Gesamtgewicht - bevorzugt in Mengen von 0,1 bis 20 Gew.-%, mehr bevorzugt von 0,25 bis 15 Gew.-%, besonders bevorzugt von 0,5 bis 12,5 Gew.-% und insbesondere von 1 bis 10 Gew.-% zugegeben werden.

Geeignete amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,
(i) 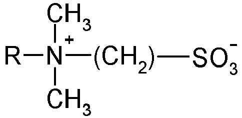
(ii) 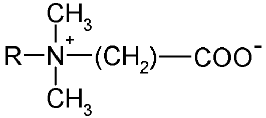
(iii) 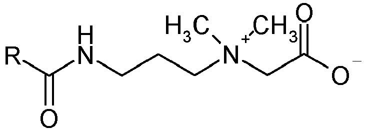
(iv) 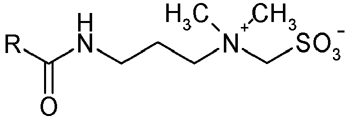
(v)

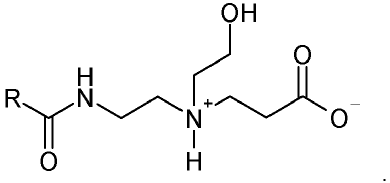

Besonders geeignete amphotere/zwitterionische Tenside sind Alkylamidoalkylbetaine und/oder Alkylampho(di)acetate der zuvor genannten Formeln (i) bis (v).

Zu den insbesondere geeigneten amphoteren/zwitterionischen Tensiden zählen die unter der INCI-Bezeichnung bekannten Tenside Cocamidopropylbetain und Disodium Cocoamphodiacetate.

In einer besonders bevorzugten Ausführungsform der Erfindung enthalten die Haarbehandlungsmittel, welche als Haarreinigungsmittel konfektioniert werden, anionische und amphotere Tenside aus den zuvor genannten Gruppen. Besonders milde Zubereitungen können erhalten werden, wenn die Reinigungszusammensetzungen mindestens ein anionisches Tensid und mindestens ein amphoteres/zwitterionisches Tensid aus den zuvor genannten Gruppen in einem Verhältnis von 5 : 1 bis 1 : 3, bevorzugt von 4,5 : 1 bis 1 : 2 und insbesondere von 4 : 1 bis 1 : 1,5 enthalten.

Kationische Tenside werden bevorzugt in erfindungsgemäßen Haarbehandlungsmitteln eingesetzt, die Haarkonditioniermittel sind.

Sie können aber auch in Mengen von bis zu 15 Gew.-% (bezogen auf das Gesamtgewicht des Mittels) in Haarreinigungsmitteln eingesetzt werden.

Geeignete kationische Tenside sind beispielsweise quartäre Ammoniumverbindungen, Esterquats und/oder Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart®, Armocare® und Quartamin® vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.

Die kationischen Tenside können in den erfindungsgemäßen Haarbehandlungsmitteln, die als Haarkonditioniermittel konfektioniert werden, bevorzugt in einer Menge von 0,05 bis 20 Gew.-%, bezogen auf das gesamte Mittel, eingesetzt werden. Mengen von 0,1 bis 15 Gew.-% sind besonders bevorzugt.

Als zweite zwingende Komponente enthalten die erfindungsgemäßen Haarbehandlungsmittel einen Extrakt natürlich fermentierter Braunalgen Laminaria Saccharina (Zuckertang), der durch natürliche (alkoholische) Gärung mit einem Pilz erhältlich ist. Die für die erfindungsgemäßen Haarbehandlungsmittel geeignete Braunalge (Zuckertang; Laminaria Saccharina) ist eine Braunalgenart, die vorwiegend in der Ostsee und im Nordatlantik in einer Tiefe von 50 bis 250 Metern beheimatet ist.

Sie besitzt einen dünnen Stiel und langgezogene "Blätter" einer Länge von 2-3 Metern.

Aus ökologischen Gesichtspunkten stammen die Braunalgen vorzugsweise aus Quellen, die ihre nachhaltige Produktion gewährleisten, beispielsweise aus der Aufzucht in speziellen Algenfarmen in der Nordsee.

Als Ausgangsmaterial für den Braunalgenextrakt b) können sowohl die zuvor beschriebenen frischen Braunalgen oder Teile der Braunalgen (die Blätter), als auch getrocknete Algen und/oder Algenblätter, die vor der Fermentation mechanisch zerkleinert werden, eingesetzt werden.

Die natürliche (alkoholische) Gärung der Braunalgen kann mit einem Pilz, bevorzugt mit einer Hefe und insbesondere mit einer Bierhefe (*Saccharomyces Cerevisiae*) initiiert werden.

Der durch die Gärung der Braunalgen entstehende Alkohol (Ethanol) dient bevorzugt als Extraktions- und Konservierungsmittel.

Die Gärung kann bei einer Temperatur von 0 bis 80°C, bevorzugt von 5 bis 70°C und insbesondere bei einer Temperatur von 10 bis 60°C erfolgen.

In einer bevorzugten Ausführungsform wird in den erfindungsgemäßen Haarbehandlungsmitteln ein Braunalgenextrakt b) eingesetzt, der von der Firma Ocean Basis im Handel unter der Bezeichnung Lamowell®S (INCI: SACCHAROMYCES/LAMINARIA SACCHARINA FERMENT) erhältlich ist.

Der Braunalgenextrakt wird in den erfindungsgemäßen Haarbehandlungsmitteln - bezogen auf deren Gesamtgewicht - bevorzugt in einer Menge von 0,001 bis 20 Gew.-%, bevorzugt von 0,005 bis 17,5 Gew.-%, mehr bevorzugt von 0,01 bis 15 Gew.-% und insbesondere von 0,1 bis 10 Gew.-% eingesetzt.

Der durch natürliche Fermentation erhältliche Braunalgenextrakt b) gemäß Anspruch 1 weist gegenüber herkömmlichen Braunalgenextrakten auf wässrig-alkoholischer Basis den Vorteil auf, dass er neben wasserlöslichen Wirkstoffen auch einen erhöhten Anteil fettlöslicher Wirkstoffe aus den Braunalgen enthält.

Die fettlöslichen Wirkstoffe aus der Braunalge, welche den Haaren und/oder der Kopfhaut vorteilhafte Eigenschaften verleihen, müssen dem jeweiligen Haarbehandlungsmittel daher nicht separat hinzugefügt und gegebenenfalls darin stabilisiert werden, wodurch sich die Herstellung des jeweiligen Haarbehandlungsmittels vereinfacht.

Der Braunalgenextrakt b) gemäß Anspruch 1 entfaltet demnach eine "Breitbandwirkung", denn es wurde gefunden, dass er in einer tensidhaltigen Grundlage sensibilisierte Kopfhaut beruhigen und den Haaren Glanz, Glätte und Weichheit verleihen kann.

In einer bevorzugten Ausführungsform wird ein erfindungsgemäßes Haarbehandlungsmittel als Haarshampoo konfektioniert und enthält - bezogen auf sein Gesamtgewicht - bevorzugt:
- 0,5 bis 25 Gew.-% eines anionischen Tensids,
- 0,1 bis 20 Gew.-% eines amphoteren/zwitterionischen Tensids und
- 0,001 bis 20 Gew.-% eines Extrakts natürlich fermentierter Braunalgen b). gemäß Anspruch 1.
Innerhalb dieser Ausführungsform ist es bevorzugt, wenn das Haarshampoo (bezogen auf sein Gesamtgewicht):
- 1 bis 20 Gew.-% Ethercarbonsäuren, Acylsarcoside, Sulfobernsteinsäuremono- und/oder - dialkylester, Alpha-Olefinsulfonate, Alkylsulfate und/oder Alkylpolyglykolethersulfatsalze,
- 0,25 bis 15 Gew.-% Alkylbetaine, Sulfobetaine, Alkylamidoalkylbetaine, Alkylamidoalkylsulfobetaine und/oder Alkylamphodiacetate und
- 0,005 bis 17,5 Gew.-% eines Extrakts natürlich fermentierter Braunalgen b) gemäß Anspruch 1

Innerhalb dieser Ausführungsform ist es mehr bevorzugt, wenn das Haarshampoo (bezogen auf sein Gesamtgewicht):
- 2 bis 17,5 Gew.-% geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten aufweisen und/oder geradkettige oder verzweigte Alkylsulfonate, die einen Alkylrest mit 8 bis 18 und insbesondere mit 10 bis 16 C-Atomen aufweisen,
- 0,5 bis 12,5 Gew.-% C₈-C₁₈-Alkylbetaine, C₈-C₁₈-Alkylamido-C₁-C₄-alkylbetaine, und/oder C₈-C₁₈-Alkylamphodiacetate und
- 0,01 bis 15 Gew.-% eines Extrakts natürlich fermentierter Braunalgen b) gemäß Anspruch 1 enthält.

Innerhalb dieser Ausführungsform ist es ganz besonders bevorzugt, wenn das Haarshampoo (bezogen auf sein Gesamtgewicht):
- 3 bis 15 Gew.-% Natrium-, Magnesium und/oder Triethanolaminsalze linearer oder verzweigter Lauryl-, Tridecyl- und/oder Myristylsulfate mit einem Ethoxylierungsgrad von 2 bis 4,
- 0,5 bis 12,5 Gew.-% Cocamidopropylbetain und/oder Disodium Cocoamphodiacetate und
- 0,1 bis 10 Gew.-% eines Extrakts natürlich fermentierter Braunalgen b) gemäß Anspruch 1 enthält.

In einer weiteren bevorzugten Ausführungsform wird ein erfindungsgemäßes Haarbehandlungsmittel als Haarspülung oder Haarkur konfektioniert und enthält - bezogen auf sein Gesamtgewicht - bevorzugt:
- 0,05 bis 20 Gew.-% quartäre Ammoniumverbindungen, Esterquats und/oder Amidoamine und
- 0,001 bis 20 Gew.-% eines Extrakts natürlich fermentierter Braunalgen b) gemäß Anspruch 1.
Innerhalb dieser Ausführungsform ist es besonders bevorzugt, wenn die Haarspülung oder die Haarkur (bezogen auf ihr Gesamtgewicht):
- 0,1 bis 15 Gew.-% Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Stearamidopropyldimethylamin, Behenoyl PG-Trimoniumchlorid und/oder die unter den INCI-Bezeichnungen bekannten Tenside Quaternium-27, Quaternium-83 und Quaternium-87, und
- 0,1 bis 10 Gew.-% eines Extrakts natürlich fermentierter Braunalgen b) gemäß Anspruch 1 enthält.

Es wurde gefunden, dass die die Kopfhaut beruhigende Wirkung des Braunalgenextrakts b) gemäß Anspruch 1 in den erfindungsgemäßen Haarbehandlungsmitteln signifikant gesteigert werden kann, wenn den jeweiligen Haarbehandlungsmitteln - bezogen auf ihr Gesamtgewicht - zusätzlich 0,01 bis 10 Gew.-%, bevorzugt 0,05 bis 7,5 Gew.-% und insbesondere 0,1 bis 5 Gew.-% mindestens eines Wirkstoffs aus der Gruppe der Vitamine, Vitaminderivate, Vitaminvorstufen, der von b) verschiedenen Pflanzenextrakte, Allantoin und/oder Glycerin zugesetzt werden.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.

Vitamin B: zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt.
- Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
- Vitamin B₆ (Pyridoxin sowie Pyridoxamin und Pyridoxal).

Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol).

Vitamin F: unter dem Begriff "Vitamin F" werden üblicherweise essentielle Fettsäuren, insbesondere Linolsäure, Linolensäure und Arachidonsäure, verstanden.

Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäßen Haarbehandlungsmittel können bevorzugt Vitamine, Provitamine und Vitaminvorstufen aus den Gruppen A, B, E und H enthalten.

Insbesondere bevorzugt sind Nicotinsäureamid, Biotin, Pantolacton und/oder Panthenol.

Unter geeigneten Pflanzenextrakten, die sich von den Braunalgenextrakten b) unterscheiden, sind Extrakte zu verstehen, die aus allen Teilen einer Pflanze hergestellt werden können. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.

Besonders bevorzugt für die Verwendung in den erfindungsgemäßen Haarbehandlungsmitteln sind die Extrakte aus Grünem Tee, Brennessel, Hamamelis, Kamille, Aloe Vera, Ginseng, Echinacea purpurea, Olea europea und/oder Boerhavia Diffusa-Wurzeln.

Mehr bevorzugt für die Verwendung in den erfindungsgemäßen Haarbehandlungsmitteln sind die Extrakte aus Grünem Tee, Kamille, Aloe Vera, Echinacea purpurea und/oder Boerhavia Diffusa-Wurzeln und insbesondere bevorzugt ist Boerhavia Diffusa-Wurzelextrakt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

In einer weiteren bevorzugten Ausführungsform wird ein erfindungsgemäßes Haarbehandlungsmittel als Haarshampoo konfektioniert und enthält - bezogen auf sein Gesamtgewicht - bevorzugt:
- 0,5 bis 25 Gew.-% eines anionischen Tensids,
- 0,1 bis 20 Gew.-% eines amphoteren/zwitterionischen Tensids,
- 0,001 bis 20 Gew.-% eines Extrakts natürlich fermentierter Braunalgen b) gemäß Anspruch 1 und
- 0,01 bis 10 Gew.-% mindestens eines Wirkstoffs aus der Gruppe der Vitamine, Vitaminderivate, Vitaminvorstufen, der von b) verschiedenen Pflanzenextrakte, Allantoin
und/oder Glycerin.

Innerhalb dieser Ausführungsform ist es bevorzugt, wenn das Haarshampoo (bezogen auf sein Gesamtgewicht):
- 1 bis 20 Gew.-% Ethercarbonsäuren, Acylsarcoside, Sulfobernsteinsäuremono- und/oder - dialkylester, Alpha-Olefinsulfonate, Alkylsulfate und/oder Alkylpolyglykolethersulfatsalze,
- 0,25 bis 15 Gew.-% Alkylbetaine, Sulfobetaine, Alkylamidoalkylbetaine, Alkylamidoalkylsulfobetaine und/oder Alkylamphodiacetate,
- 0,005 bis 17,5 Gew.-% eines Extrakts natürlich fermentierter Braunalgen b) gemäß Anspruch 1 und
- 0,05 bis 7,5 Gew.-% eines Vitamins, Provitamins und/oder einer Vitaminvorstufe aus den Gruppen A, B, E und H, eines Extrakte aus Grünem Tee, Brennessel, Hamamelis, Kamille, Aloe Vera, Ginseng, Echinacea purpurea, Olea europea und/oder Boerhavia Diffusa-Wurzeln, Allantoin und/oder Glycerin enthält.

Innerhalb dieser Ausführungsform ist es mehr bevorzugt, wenn das Haarshampoo (bezogen auf sein Gesamtgewicht):
- 2 bis 17,5 Gew.-% geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten aufweisen und/oder geradkettige oder verzweigte Alkylsulfonate, die einen Alkylrest mit 8 bis 18 und insbesondere mit 10 bis 16 C-Atomen aufweisen,
- 0,5 bis 12,5 Gew.-% C₈-C₁₈-Alkylbetaine, C₈-C₁₈-Alkylamido-C₁-C₄-alkylbetaine, und/oder C₈-C₁₈-Alkylamphodiacetate,
- 0,01 bis 15 Gew.-% eines Extrakts natürlich fermentierter Braunalgen b) gemäß Anspruch 1 und
- 0,1 bis 5 Gew.-% Nicotinsäureamid, Biotin, Pantolacton, Panthenol, einen Extrakt aus Grünem Tee, Kamille, Aloe Vera, Echinacea purpurea und/oder Boerhavia Diffusa-Wurzeln, Allantoin und/oder Glycerin enthält.

Innerhalb dieser Ausführungsform ist es ganz besonders bevorzugt, wenn das Haarshampoo (bezogen auf sein Gesamtgewicht):
- 3 bis 15 Gew.-% Natrium-, Magnesium und/oder Triethanolaminsalze linearer oder verzweigter Lauryl-, Tridecyl- und/oder Myristylsulfate mit einem Ethoxylierungsgrad von 2 bis 4,
- 0,5 bis 12,5 Gew.-% Cocamidopropylbetain und/oder Disodium Cocoamphodiacetate,
- 0,1 bis 10 Gew.-% eines Extrakts natürlich fermentierter Braunalgen b) gemäß Anspruch 1 und
- 0,1 bis 5 Gew.-% Nicotinsäureamid, Pantolacton, Panthenol, Boerhavia Diffusa-Wurzelextrakt, Allantoin und/oder Glycerin enthält.

In einer weiteren bevorzugten Ausführungsform wird ein erfindungsgemäßes Haarbehandlungsmittel als Haarspülung oder Haarkur konfektioniert und enthält - bezogen auf sein Gesamtgewicht - bevorzugt:
- 0,05 bis 20 Gew.-% quartäre Ammoniumverbindungen, Esterquats und/oder Amidoamine,
- 0,001 bis 20 Gew.-% eines Extrakts natürlich fermentierter Braunalgen b) gemäß Anspruch 1 und
- 0,05 bis 7,5 Gew.-% eines Vitamins, Provitamins und/oder einer Vitaminvorstufe aus den Gruppen A, B, E und H, eines Extrakte aus Grünem Tee, Brennessel, Hamamelis, Kamille, Aloe Vera, Ginseng, Echinacea purpurea, Olea europea und/oder Boerhavia Diffusa- Wurzeln, Allantoin und/oder Glycerin.

Innerhalb dieser Ausführungsform ist es besonders bevorzugt, wenn die Haarspülung oder die Haarkur (bezogen auf ihr Gesamtgewicht):
- 0,1 bis 15 Gew.-% Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Stearamidopropyldimethylamin, Behenoyl PG-Trimoniumchlorid und/oder die unter den INCI-Bezeichnungen bekannten Tenside Quaternium-27, Quaternium-83 und Quaternium-87,
- 0,1 bis 10 Gew.-% eines Extrakts natürlich fermentierter Braunalgen b) gemäß Anspruch 1 und
- 0,1 bis 5 Gew.-% Nicotinsäureamid, Biotin, Pantolacton, Panthenol, einen Extrakt aus Grünem Tee, Kamille, Aloe Vera, Echinacea purpurea und/oder Boerhavia Diffusa-Wurzeln, Allantoin und/oder Glycerin enthält.

Neben den zuvor genannten Wirkstoffen können die erfindungsgemäßen Haarbehandlungsmittel noch eine Reihe weiterer Wirkstoffe enthalten, die ihnen vorteilhafte Eigenschaften verleihen.

Zu den bevorzugten fakultativen Wirkstoffen, die in den erfindungsgemäßen Haarbehandlungsmitteln eingesetzt werden können, zählen beispielsweise:
- kationische Polymere, die in den jeweiligen Haarbehandlungsmitteln (bezogen auf ihr Gesamtgewicht) bevorzugt in einer Menge von 0,01 bis 10 Gew.-%, mehr bevorzugt von 0,05 bis 7,5 Gew.-% und insbesondere von 0,1 bis 5 Gew.-% eingesetzt werden und
- Antischuppenwirkstoffe, die in den jeweiligen Haarbehandlungsmitteln (bezogen auf ihr Gesamtgewicht) bevorzugt in einer Menge von 0,01 bis 10 Gew.-%, mehr bevorzugt von 0,025 bis 7,5 Gew.-%, besonders bevorzugt von 0,05 bis 5 Gew.-% und insbesondere von 0,075 bis 3 Gew.-% eingesetzt werden.

Geeignete kationische Polymere sind beispielsweise:
- quaternisierte Cellulose-Derivate, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Be zeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Weitere geeignete kationischen Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.

Besonders bevorzugte kationische Polymere, die in den erfindungsgemäßen Haarbehandlungsmitteln eingesetzt werden können, sind quaternisierte Cellulosepolymere, kationische Guarderivate und/oder kationische Polymere auf Acrylsäure(derivat)basis, die insbesondere ausgewählt sind aus den unter den INCI-Bezeichnungen bekannten Polymeren Polyquaternium-6, Polyquaternium-7, Polyquaternium-10, Polyquaternium-37 und/oder Polyquaternium-67.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

Bevorzugt sind Climbazol, Zink Pyrithion und Piroctone Olamine.

In einer weiteren bevorzugten Ausführungsform wird ein erfindungsgemäßes Haarbehandlungsmittel als Haarshampoo konfektioniert und enthält - bezogen auf sein Gesamtgewicht - bevorzugt:
- 0,5 bis 25 Gew.-% eines anionischen Tensids,
- 0,1 bis 20 Gew.-% eines amphoteren/zwitterionischen Tensids,
- 0,001 bis 20 Gew.-% eines Extrakts natürlich fermentierter Braunalgen b) gemäß Anspruch 1 und
- 0,01 bis 10 Gew.-% mindestens eines kationischen Polymers und/oder mindestens eines Antischuppenwirkstoffs.

Innerhalb dieser Ausführungsform ist es bevorzugt, wenn das Haarshampoo (bezogen auf sein Gesamtgewicht):
- 1 bis 20 Gew.-% Ethercarbonsäuren, Acylsarcoside, Sulfobernsteinsäuremono- und/oder - dialkylester, Alpha-Olefinsulfonate, Alkylsulfate und/oder Alkylpolyglykolethersulfatsalze,
- 0,25 bis 15 Gew.-% Alkylbetaine, Sulfobetaine, Alkylamidoalkylbetaine, Alkylamidoalkylsulfobetaine und/oder Alkylamphodiacetate,
- 0,005 bis 17,5 Gew.-% eines Extrakts natürlich fermentierter Braunalgen b) gemäß Anspruch 1 und
- 0,05 bis 7,5 Gew.-% mindestens eines kationischen Polymers und/oder 0,05 bis 5 Gew.-% Piroctone Olamine, Climbazol, Zink Pyrithion, Ketoconazole, Salicylsäure, Schwefel, Selensulfid, Teerpräparate, Undecensäurederivate, Klettenwurzelextrakt, Pappelextrakt, Brennesselextrakt, Walnussschalenextrakt, Birkenextrakt, Weidenrindenextrakt, Rosmarinextrakt und/oder Arnikaextrakt enthält.

Innerhalb dieser Ausführungsform ist es ganz besonders bevorzugt, wenn das Haarshampoo (bezogen auf sein Gesamtgewicht):
- 3 bis 15 Gew.-% Natrium-, Magnesium und/oder Triethanolaminsalze linearer oder verzweigter Lauryl-, Tridecyl- und/oder Myristylsulfate mit einem Ethoxylierungsgrad von 2 bis 4,
- 0,5 bis 12,5 Gew.-% Cocamidopropylbetain und/oder Disodium Cocoamphodiacetate,
- 0,1 bis 10 Gew.-% eines Extrakts natürlich fermentierter Braunalgen b) gemäß Anspruch 1 und
- 0,1 bis 5 Gew.-% mindestens eines der kationischen Polymere Polyquaternium-6, Polyquaternium-7, Polyquaternium-10, Polyquaternium-37, Polyquaternium-67und/oder 0,075 bis 3 Gew.-% Piroctone Olamine, Climbazol und/oder Zink Pyrithion enthält.

In einer weiteren bevorzugten Ausführungsform wird ein erfindungsgemäßes Haarbehandlungsmittel als Haarspülung oder Haarkur konfektioniert und enthält - bezogen auf sein Gesamtgewicht - bevorzugt:
- 0,05 bis 20 Gew.-% quartäre Ammoniumverbindungen, Esterquats und/oder Amidoamine,
- 0,001 bis 20 Gew.-% eines Extrakts natürlich fermentierter Braunalgen b) gemäß Anspruch 1 und
- 0,05 bis 7,5 Gew.-% eines kationischen Polymers.

Innerhalb dieser Ausführungsform ist es besonders bevorzugt, wenn die Haarspülung oder die Haarkur (bezogen auf ihr Gesamtgewicht):
- 0,1 bis 15 Gew.-% Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Stearamidopropyldimethylamin, Behenoyl PG-Trimoniumchlorid und/oder die unter den INCI-Bezeichnungen bekannten Tenside Quaternium-27, Quaternium-83 und Quaternium-87,
- 0,1 bis 10 Gew.-% eines Extrakts natürlich fermentierter Braunalgen b) gemäß Anspruch 1 und
- 0,1 bis 5 Gew.-% mindestens eines kationischen Polymers aus der Gruppe der quaternisierten Cellulosepolymere, kationischen Guarderivate und/oder kationischen Polymere auf Acrylsäure(derivat)basis enthält.

Die erfindungsgemäßen Haarbehandlungsmittel können bevorzugt als Haarshampoo und/oder Haarkur (Haarspülung) konfektioniert werden.

Innerhalb dieser Konfektionierungsformen können die Haarbehandlungsmittel neben den zuvor beschriebenen Komponenten weitere Komponenten enthalten, die dem Mittel weitere vorteilhafte Eigenschaften verleihen.

Zu den weiteren fakultativen Komponenten zählen beispielsweise
- nichtionische Tenside und/oder nichtionische Emulgatoren,
- Öl- und/oder Fettkomponenten,
- Proteinhydrolysate und/oder
- Feuchthaltemittel.

Geeignete nichtionische Tenside und/oder nichtionische Emulgatoren können den erfindungsgemäßen Haarbehandlungsmitteln - bezogen auf deren Gesamtgewicht - bevorzugt in einer Menge von 0,1 bis 30 Gew.-%, bevorzugt 0,5 bis 20 Gew.-% und insbesondere 1 bis 15 Gew.-% zugesetzt werden.

Zu den geeigneten nichtionischen Tensiden/Emulgatoren zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Für den Fall, dass ein nichtionisches Tensid in den Haarbehandlungsmitteln eingesetzt wird, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Weiterhin bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

Geeignete Öl- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

Als synthetische Öle kommen Silikonverbindungen in Betracht.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Es ist daher erstrebenswert, in den kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopolymeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol® OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12, Cetiol® HE oder Cutina® MD.

Die Einsatzmenge aller geeigneter Öl- und/oder Fettkomponenten in den erfindungsgemäßen Haarbehandlungsmitteln beträgt bevorzugt 0,01 - 50 Gew.% bezogen auf das gesamte Mittel. Mehr bevorzugt sind 0,05 - 30 Gew.% und besonders bevorzugt 0,1 - 20 Gew.%, bezogen auf das gesamte Mittel.

Geeignete Proteinhydrolysate sind Produktgemische, die durch sauer, basisch oder enzymatisch katalysierten Abbau von Proteinen (Eiweißen) erhalten werden.

Es können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis. Die Proteinhydrolysate und deren Derivate können bevorzugt in Mengen von 0,01 - 10 Gew.-%, bezogen auf das gesamte Haarbehandlungsmittel, eingesetzt werden. Mengen von 0,1 bis 5 Gew.-% und insbesondere 0,1 bis 3 Gew.-%, sind ganz besonders bevorzugt.

Geeignete Feuchthaltemittel bzw. Penetrationshilfsstoffe und/ oder Quellmittel, die den erfindungsgemäßen Haarbehandlungsmitteln zugesetzt werden können, sind beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Die Feuchthaltemittel können in den erfindungsgemäßen Zusammensetzungen - bezogen auf deren Gesamtgewicht - in Mengen von 0,01 bis 10 Gew.-%, bevorzugt in Mengen von 0,05 bis 5 Gew.-% und insbesondere in Mengen von 0,1 bis 3 Gew.-% eingesetzt werden.

Weitere Wirk-, Hilfs- und Zusatzstoffe, die in den erfindungsgemäßen Haarbehandlungsmitteln eingesetzt werden können, sind beispielsweise:
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Cholesterin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konsistenzgeber wie Zuckerester, Polyolester oder Polyolalkylether,
- Konservierungsmittel, wie beispielsweise Natriumbenzoat oder Salicylsäure,
- Viskositätsreglerwie Salze (NaCl).

Die erfindungsgemäßen Haarbehandlungsmittel weisen bevorzugt einen pH-Wert im Bereich von 1 bis 8, bevorzugt von 2 bis 7 und insbesondere von 3 bis 6, auf.

Die erfindungsgemäßen Haarbehandlungsmittel weisen hervorragende Eigenschaften in der Anwendung auf den Haaren auf.

Sie verleihen den damit behandelten Haaren mehr Glanz, Glätte und Weichheit.

Daneben vermögen sie sensibilisierte und/oder gereizte Kopfhaut zu beruhigen, sowie die Anzeichen trockener, rauer, rissiger, geröteter und/oder schuppiger Kopfhaut zu lindern.

Durch die Verwendung des Braunalgenferments b) konnten Haarbehandlungsmittel mit "Breitbandwirkung" hergestellt werden.

Ein zweiter Gegenstand der Erfindung ist die Verwendung des erfindungsgemäßen Mittels zur Beruhigung empfindlicher Kopfhaut und/oder durch chemische Haarbehandlungen geschädigter und/oder gereizter Kopfhaut während der Haarreinigung und/oder der Haarpflege.

### Beispiele:

Die erfindungsgemäßen Haarbehandlungsmittel wurden hinsichtlich ihrer die Kopfhaut beruhigenden Fähigkeit untersucht

Der Effekt einer Tensidlösung (Natriumlaurylethersulfat) wurde an Epidermismodellen in vitro untersucht, indem die Zellvitalität und der Gehalt des Entzündungsmediators (Interleukin-1 alpha (IL-1α)) bestimmt wurde.

Es wurden die folgenden Epidermismodelle untersucht:
a) unbehandelt,
b) Epidermismodelle, die mit einer 0,01%igen Natriumlaurylethersulfatlösung behandelt wurden,
c) Epidermismodelle, die mit Cortison behandelt wurden,
d) Epidermismodelle, die mit Cortison und einer 0,01%igen Natriumlaurylethersulfatlösung behandelt wurden,
e) Epidermismodelle, die mit einer 0,01%igen Natriumlaurylethersulfatlösung und dem Braunalgenferment b) behandelt wurden.

### 1) Zellvitalität nach der Behandlung

Die folgenden Werte beziehen sich auf die unbehandelte Epidermismodelle
a) (=100% Vitalität):
b) 75% Zellvitalität,
c) 90% Zellvitalität,
d) 82% Zellvitalität,
e) 90% Zellvitalität.

### 2) IL-1 alpha-Konzentration nach der Behandlung (pg/ml)

a) 95
b) 220
c) 100
d) 180
e) 90

Die Ergebnisse zeigen, dass die Zellvitalität der Keratinozyten in den Epidermismodellen nach der Behandlung mit der erfindungsgemäßen Kombination aus einem Tensid (Natriumlaurylethersulfat) und einem Braunalgenferment b) hoch ist, und dass die IL-1 alpha-Konzentration (als Maß für die Entzündung der Kopfhaut) nach der Behandlung mit der erfindungsgemäßen Kombination im Bereich der unbehandelten Epidermismodelle liegt.

Es wurden die folgenden Ausführungsbeispiele hergestellt (die Mengenangaben beziehen sich - sofern nicht anders angegeben - auf Gew.-%):

### 1) Haarshampoos

| | **1** | **2** | **3** | **4** |
|---|---|---|---|---|
| **Texapon®**¹ **N70** | 15 | 15 | 12 | 18 |
| **Dehyton®² K** | 10 | - | - | 15 |
| **Dehyton®³ G** | - | 15 | 12 | - |
| **Plantacare®⁴ 818 UP** | 4 | - | 2 | - |
| **D-Panthenol** | 0,5 | 0,2 | 0,2 | 0,3 |
| **Nicotinsäureamid** | 0,3 | 0,1 | 0,15 | 0,2 |
| **Mediacalm®⁵** | - | 2 | 1 | - |
| **Allantoin** | - | 0,1 | 0,2 | 0,1 |
| **Glycerin** | - | 1 | 0,5 | 2 |
| **Polymer JR®⁶ 400** | 0,3 | 0,5 | - | 0,3 |
| **Polyquaternium-7** | - | - | 0,5 | 0,3 |
| **Dow Corning®⁷ 200** | - | 0,5 | 0,2 | 0,1 |
| **Citronensäure** | 0,5 | 0,6 | 0,2 | 0,4 |
| **Arlypon®⁸ F** | 1,2 | 1 | 1 | 1,3 |
| **NaCl** | 1,3 | 1 | 0,7 | 1 |
| **Lamowell®⁹ S** | 1 | 2 | 1,5 | 2 |
| **Zink Pyrithion** | - | 0,5 | 0,2 | 0,3 |
| **Konservierungsmittel, Parfum** | q.s. | q.s. | q.s. | q.s. |
| **Wasser** | ad 100 | ad 100 | ad 100 | ad 100 |

| | | | | |
|---|---|---|---|---|
| In den zuvor genannten Haarshampoos wurden die folgenden Handelsprodukte eingesetzt: 1 INCI-Bezeichnung: Sodium Laureth Sulfate; AS 68-73%; Cognis, 2 INCI-Bezeichnung: Cocamidopropyl Betaine; AS 29-32%; Cognis, 3 INCI-Bezeichnung: Disodium Cocoamphodiacetate; AS 30%; Cognis, 4 INCI-Bezeichnung: Coco Glucoside; AS 51-53%; Cognis, 5 INCI-Bezeichnung: Aqua, Butylene Glycol, Boerhavia Diffusa Root Extract; Silab 6 INCI-Bezeichnung: Polyquaternium-10; Dow 7 INCI-Bezeichnung: Dimethicone (30,000 cst); Dow Corning 8 INCI-Bezeichnung: Laureth-2; Cognis 9 INCI-Bezeichnung: Saccharomyces/Laminaria Saccharina Ferment; Ocean Basis | | | | |

### 2) Haarspülungen

| | **1** | **2** | **3** |
|---|---|---|---|
| **Lanette®¹⁰ O** | 6,25 | 5,75 | 7 |
| **Varisoft®¹¹ W575 PG** | 0,75 | 1 | - |
| **Cutina®¹² AGS** | 1 | - | 1,2 |
| **Shea Butter** | 1,5 | - | 1 |
| **Aprikosenkernöl** | - | 1 | - |
| **Pantolacton** | 0,5 | 0,2 | 0,2 |
| **Rambutanöl** | 0,5 | - | 0,1 |
| **Quartamin®¹³ BTC-131** | 1,5 | - | 1,2 |
| **Dehyquart®¹⁴ F75** | 0,75 | - | 0,5 |
| **Dehyquart®¹⁵ A CA** | - | 1 | - |
| **Cosmedia®¹⁶ CTH** | 0,4 | 0,5 | 0,6 |
| **Glycerin** | 0,1 | 0,3 | 0,5 |
| **Dow Corning®⁷ 200** | 1 | 1,2 | 1 |
| **Milchsäure** | 0,1 | 0,4 | 0,15 |
| **Lamowell®⁹ S** | 5 | 3 | 3,5 |
| **Zink Pyrithion** | - | 0,25 | 0,2 |
| **Konservierungsmittel, Parfum** | q.s. | q.s. | q.s. |
| **Wasser** | ad 100 | ad 100 | ad 100 |

| | | | |
|---|---|---|---|
| In den zuvor genannten Haarspülungen wurden die folgenden Handelsprodukte eingesetzt: 10 INCI-Bezeichnung: Cetearyl Alcohol; Cognis, 11 INCI-Bezeichnung: Quaternium-87, Propylene Glycol; Evonik, 12 INCI-Bezeichnung: Glycol Distearate; Cognis, 13 INCI-Bezeichnung: Behenoyl PG-Trimonium Chloride; AS 50%; Kao, 14 INCI-Bezeichnung: Distearoylethyl Hydroxyethylmonium Methosulfate, Cetearyl Alcohol, AS 65-72%; Cognis, 15 INCI-Bezeichnung: Aqua Cetrimonium Chloride, AS 24-26%; Cognis, 16 INCI-Bezeichnung: Polyquaternium-37, Propylene Glycol Dicaprylate/Dicaprate, PPG-1Trideceth-6, Cognis | | | |

## Patentansprüche

1. Haarbehandlungsmittel, enthaltend in einem kosmetischen Träger
a) 0,1 bis 40 Gew.-% mindestens eines Tensids, ausgewählt aus der Gruppe der anionischen, amphoteren/zwitterionischen und/oder kationischen Tenside, und
b) einen Extrakt der natürlich fermentierten Braunalgenart *Laminaria Saccharina (Zuckertang),* der durch natürliche (alkoholische) Gärung mit einem Pilz erhältlich ist,
wobei sich die Mengenangaben auf das Gesamtgewicht des Haarbehandlungsmittels beziehen.

2. Haarbehandlungsmittel nach Anspruch 1, **dadurch gekennzeichnet, dass** der Extrakt b) durch alkoholische Gärung der Braunalgenart *Laminaria Saccharina (Zuckertang)* mit einer Bierhefe (*Saccharomyces Cerevisiae*) erhalten wird.

3. Haarbehandlungsmittel nach einem der Ansprüche 1 oder 2, **dadurch gekennzeichnet, dass** es - bezogen auf sein Gesamtgewicht - 0,001 bis 20 Gew.-%, bevorzugt 0,005 bis 17,5 Gew.-%, mehr bevorzugt 0,01 bis 15 Gew.-% und insbesondere 0,1 bis 10 Gew.-% des Braunalgenextrakts b) enthält.

4. Haarbehandlungsmittel nach einem der Ansprüche 1 bis 3, **dadurch gekennzeichnet, dass** es mindestens ein kationisches Tensid aus der Gruppe der quartären Ammoniumverbindungen, der Esterquats und/oder der Amidoamine und/oder mindestens ein anionisches Tensid aus der Gruppe der Alkyl(ether)sulfate, der Ethercarbonsäuren, der Sulfobernsteinsäuremono- und/oder -dialkylester, der Olefinsulfonate und/oder der Acylsarcoside und/oder mindestens ein amphoteres/zwitterionisches Tensid aus der Gruppe der Alkylamidoalkylbetaine und/oder der Alkylampho(di)acetate enthält.

5. Haarbehandlungsmittel nach einem der Ansprüche 1 bis 4, **dadurch gekennzeichnet, dass** es bezogen auf sein Gesamtgewicht - 0,01 bis 10 Gew.-%, bevorzugt 0,05 bis 7,5 Gew.-% und insbesondere 0,1 bis 5 Gew.-% mindestens eines Vitamins, eines von b) verschiedenen Pflanzenextrakts, Allantoin und/oder Glycerin enthält.

6. Haarbehandlungsmittel nach Anspruch 5, **dadurch gekennzeichnet, dass** es Panthenol, Pantolacton, Nicotinsäureamid, Biotin, Boerhavia Diffusa-Wurzelextrakt, Allantoin und/oder Glycerin enthält.

7. Haarbehandlungsmittel nach einem der Ansprüche 1 bis 6, **dadurch gekennzeichnet, dass** es - bezogen auf sein Gesamtgewicht - 0,01 bis 10 Gew.-%, bevorzugt 0,05 bis 7,5 Gew.-% und insbesondere 0,1 bis 5 Gew.-% eines kationischen Polymeren enthält.

8. Haarbehandlungsmittel nach einem der Ansprüche 1 bis 7, **dadurch gekennzeichnet, dass** es - bezogen auf sein Gesamtgewicht - 0,01 bis 10 Gew.-%, bevorzugt 0,025 bis 7,5 Gew.-%, mehr bevorzugt 0,05 bis 5 Gew.-% und insbesondere 0,075 bis 3 Gew.-% mindestens eines Antischuppenwirkstoffs enthält.

9. Haarbehandlungsmittel nach Anspruch 8, **dadurch gekennzeichnet, dass** es als Antischuppenwirkstoff Piroctone Olamine, Climbazol und/oder Zink Pyrithion enthält.

## Claims

1. A hair treatment agent, containing in a cosmetic carrier
a) from 0.1 to 40 wt.% of at least one surfactant, selected from the group of anionic, amphoteric/zwitterionic and/or cationic surfactants, and
b) an extract of the naturally fermented brown algae species *Laminaria Saccharina (sugar kelp),* which can be obtained by natural (alcoholic) fermentation with a fungus,
wherein the quantities stated relate to the total weight of the hair treatment agent.

2. The hair treatment agent according to claim 1, **characterized in that** the extract b) is obtained by alcoholic fermentation of the brown algae species *Laminaria Saccharina (sugar kelp)* with a yeast *(Saccharomyces Cerevisiae).*

3. The hair treatment agent according to one of claims 1 or 2, **characterized in that** it contains, based on the total weight thereof, from 0.001 to 20 wt.%, preferably from 0.005 to 17.5 wt.%, more preferably from 0.01 to 15 wt.%, and in particular from 0.1 to 10 wt.%, of the brown algae extract b).

4. The hair treatment agent according to one of claims 1 to 3, **characterized in that** it contains at least one cationic surfactant from the group of quaternary ammonium compounds, esterquats and/or amidoamines and/or at least one anionic surfactant from the group of alkyl (ether) sulfates, ether carboxylic acids, sulfosuccinic acid monoalkyl and/or dialkyl esters, olefin sulfonates and/or acyl sarcosides and/or at least one amphoteric/zwitterionic surfactant from the group of alkylamido alkyl betaines and/or alkyl ampho(di)acetates.

5. The hair treatment agent according to one of claims 1 to 4, **characterized in that** it contains, based on the total weight thereof, from 0.01 to 10 wt.%, preferably from 0.05 to 7.5 wt.%, and in particular from 0.1 to 5 wt.%, of at least one vitamin, a plant extract that is different from b), allantoin and/or glycerol.

6. The hair treatment agent according to claim 5, **characterized in that** it contains panthenol, pantolactone, nicotinamide, biotin, Boerhavia diffusa root extract, allantoin and/or glycerol.

7. The hair treatment agent according to one of claims 1 to 6, **characterized in that** it contains, based on the total weight thereof, from 0.01 to 10 wt.%, preferably from 0.05 to 7.5 wt.%, and in particular from 0.1 to 5 wt.%, of a cationic polymer.

8. The hair treatment agent according to one of claims 1 to 7, **characterized in that** it contains, based on the total weight thereof, from 0.01 to 10 wt.%, preferably from 0.025 to 7.5 wt.%, more preferably from 0.05 to 5 wt.%, and in particular from 0.075 to 3 wt.%, of at least one anti-dandruff active ingredient.

9. The hair treatment agent according to claim 8, **characterized in that** it contains piroctone olamine, climbazole and/or zinc pyrithione as the anti-dandruff active ingredient.

## Revendications

1. Agent de traitement capillaire, contenant dans un support cosmétique
a) de 0,1 à 40 % en poids d'au moins un tensioactif, sélectionné dans le groupe constitué des tensioactifs anioniques, amphotères/zwitterioniques et/ou cationiques, et
b) un extrait d'algue brune naturellement fermentée de type *Laminaria Saccharina* (*laminaire sucrée*), qui peut être obtenu par la fermentation naturelle (alcoolique) avec un champignon,
dans lequel les données relatives aux quantités se rapportent au poids total de l'agent de traitement capillaire.

2. Agent de traitement capillaire selon la revendication 1, **caractérisé en ce que** l'extrait b) est obtenu par fermentation alcoolique de l'algue brune de type *Laminaria Saccharina* (*laminaire sucrée*), avec une levure de bière (*Saccharomyces Cerevisiae*).

3. Agent de traitement capillaire selon l'une des revendications 1 ou 2, **caractérisé en ce qu'**il contient - rapporté à son poids total - de 0,001 à 20 % en poids, de manière préférée de 0,005 à 17,5 % en poids, de manière davantage préférée de 0,01 à 15 % en poids et en particulier de 0,1 à 10 % en poids de l'extrait d'algue brune b).

4. Agent de traitement capillaire selon l'une des revendications 1 à 3, **caractérisé en ce qu'**il contient au moins un tensioactif cationique issu du groupe des composés d'ammonium quaternaires, des esterquats et/ou des amidoamines et/ou au moins un tensioactif anionique issu du groupe des alkyl(éther)sulfates, des acides éther-carboxyliques, des mono- et/ou dialkylester d'acide sulfosuccinique, des oléfines sulfonates et/ou des acylsarcosides et/ou au moins un tensioactif amphotère/zwitterionique issu du groupe des alkylamidoalkylbétaïnes et/ou des alkylampho(di)acétates.

5. Agent de traitement capillaire selon l'une des revendications 1 à 4, **caractérisé en ce qu'**il contient, rapporté à son poids total, de 0,01 à 10 % en poids, de manière préférée de 0,05 à 7,5 % en poids et en particulier de 0,1 à 5 % en poids d'au moins une vitamine, un extrait de plante différent de b), de l'allantoïne et/ou de la glycérine.

6. Agent de traitement capillaire selon la revendication 5, **caractérisé en ce qu'**il contient du panthénol, de la pantolactone, du nicotinamide, de la biotine, de l'extrait de racine de Boerhavia Diffusa, de l'allantoïne et/ou de la glycérine.

7. Agent de traitement capillaire selon l'une des revendications 1 à 6, **caractérisé en ce qu'**il contient, rapporté à son poids total, de 0,01 à 10 % en poids, de manière préférée de 0,05 à 7,5 % en poids et en particulier de 0,1 à 5 % en poids d'un polymère cationique.

8. Agent de traitement capillaire selon l'une des revendications 1 à 7, **caractérisé en ce qu'**il contient, rapporté à son poids total, de 0,01 à 10 % en poids, de manière préférée de 0,025 à 7,5 % en poids, de manière davantage préférée de 0,05 à 5 % en poids et en particulier de 0,075 à 3 % en poids d'au moins un principe actif antipelliculaire.

9. Agent de traitement capillaire selon la revendication 8, **caractérisé en ce qu'**il contient de la piroctone olamine, du climbazole et/ou du pyrithione de zinc en tant que principe actif antipelliculaire.
